(19) Europäisches Patentamt
European Patent Office
Office européen des brevets

(11) EP 0 484 219 B1

(12) **FASCICULE DE BREVET EUROPEEN**

(45) Date de publication et mention de la délivrance du brevet:
**29.05.1996 Bulletin 1996/22**

(51) Int. Cl.[6]: **C07C 37/00**, C07C 39/38

(21) Numéro de dépôt: **91402877.4**

(22) Date de dépôt: **28.10.1991**

(54) **Procédé de débromation de dérivés dibromés du naphtalène**

Verfahren zum Debromieren der bromierten Naphthalinverbindungen

Process for debrominating dibrominated naphthalene derivatives

(84) Etats contractants désignés:
**AT BE CH DE DK ES FR GB GR IT LI LU NL SE**

(30) Priorité: **31.10.1990 FR 9013512**

(43) Date de publication de la demande:
**06.05.1992 Bulletin 1992/19**

(73) Titulaire: **POTASSE ET PRODUITS CHIMIQUES**
**F-92400 Courbevoie (FR)**

(72) Inventeurs:
- **Jacquot, Roland**
  **F-69110 Sainte Foy les Lyon (FR)**
- **Truchet, Françoise**
  **F-69006 Lyon (FR)**

(74) Mandataire: **Woods, Geoffrey Corlett et al**
**J.A. KEMP & CO.**
**14 South Square**
**Gray's Inn**
**London WC1R 5LX (GB)**

(56) Documents cités:
**EP-A- 0 055 196** **GB-A- 380 563**
**US-A- 2 725 402**

- **SYNTHESIS, no. 10, octobre 1982, pages 876-878, Speyer/Rhein; P.N. PANDEY et al.: "Palladium-catalyzed hydrodehalogenation of haloaromatic compounds"**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Rank Xerox (UK) Business Services
2.11.7/3.4

## Description

La présente invention porte sur un procédé de débromation de dérivés dibromés du naphtalène. Plus précisément, l'invention concerne un procédé de monodébromation de dérivés dibromés du naphtalène de formule :

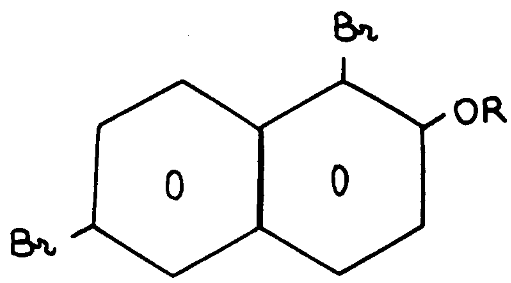

(1)

dans laquelle R représente l'hydrogène, un groupement alkyle, aryle, aralkyle ou

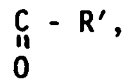

R' étant un radical alkyle, au moyen d'une hydrodébromation catalytique régio-sélective.

Les produits de réaction qui sont ainsi plus particulièrement visés par le procédé de débromation selon l'invention répondent à la formule :

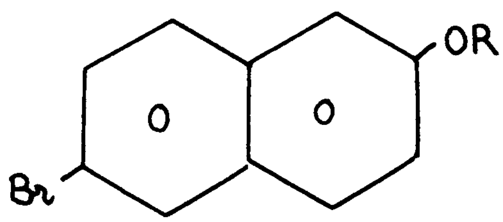

(2)

dans laquelle R a la signification donnée ci-dessus.

Les bromo-6 naphtalènes de formule (2) ci-dessus sont des produits particulièrement intéressants et importants. Ainsi, par exemple, le bromo-6 methoxy-2 naphtalène est largement utilisé pour la synthèse du naproxène ou de la nabumétone, deux produits notoirement connus pour leurs propriétés thérapeutiques anti-inflammatoires, ainsi que pour la synthèse du methallenestril, qui est un estrogène (voir à ce sujet : the Merck Index, eleventh edition, 1989, pages 1002, 1014 et 937).

Le bromo 6- hydroxy-2 naphtalène (aussi appelé bromo-6β naphtol) est, quant à lui, principalement utilisé pour la synthèse du bromo-6 méthoxy-2 naphtalène susmentionné, par alkylation au moyen de sulfate de diméthyle ou du méthanol.

Selon l'enseignement du document EP-A-179 447, les bromo-6 [hydroxy ou alkoxy]-2 naphtalènes peuvent être préparés par réduction métallique stoëchiométrique des dibromo-1,6 [hydroxy ou alkoxy]-2 naphtalènes correspondants, selon la réaction suivante :

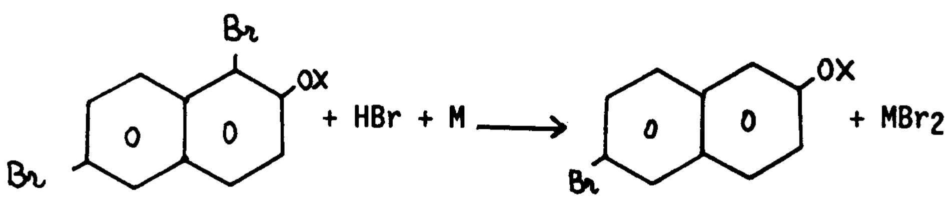

où X désigne l'hydrogène ou un radical alkyle, et M un métal réducteur tel que fer ou étain, les dérivés dibromés ci-dessus pouvant eux-mêmes être préparés simplement par bromation directe des dérivés non bromés correspondants :

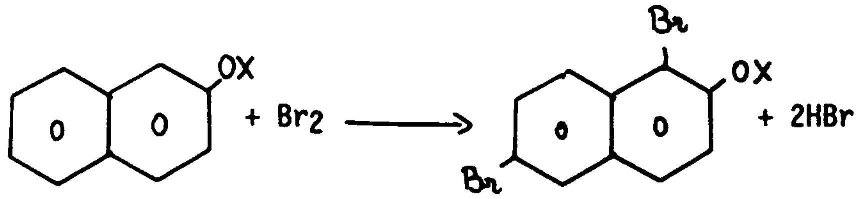

Toutefois, la réduction des dérivés dibromés du naphtalène en dérivés monobromés selon un procédé tel que décrit ci-dessus présente le désavantage, entre autres, de nécessiter une consommation importante en métal, métal qui se retrouve par ailleurs sous la forme d'un effluent perdu, difficilement valorisable, et parfois polluant, comme par exemple Fe $Br_2$.

En outre, les rendements de réaction en dérivés monobromés désirés, par un tel procédé, peuvent s'avérer insuffisants.

La présente invention a pour but de proposer un procédé de débromation de certains dérivés dibromés du naphtalène permettant d'une part d'obvier aux inconvénients susmentionnés, et d'autre part de réaliser une débromation régio-sélective, en particulier en position 1, avec un rendement élevé.

A cet effet, il est maintenant proposé un nouveau procédé de débromation de dérivés dibromés du naphtalène, ledit procédé étant caractérisé par le fait que l'on fait réagir, en masse ou dans un solvant organique acide, en présence d'un catalyseur d'hydrodébromation, (i) un dérivé dibromé du naphtalène de formule :

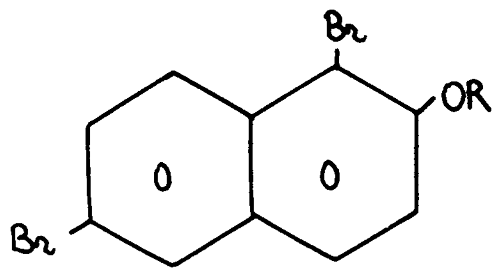

(1)

dans laquelle R représente l'hydrogène, un groupement alkyle, aryle, aralkyle ou

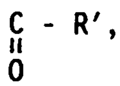

R′ étant un radical alkyle, avec (ii) de l'hydrogène moléculaire ou un composé susceptible de générer, dans le milieu de réaction, de l'hydrogène naissant.

Le procédé selon l'invention présente de nombreux avantages et une grande souplesse d'utilisation. Il permet tout d'abord d'éviter la consommation stoëchiométrique de métaux réducteurs.Par ailleurs, de façon inattendue et surprenante, il est également hautement sélectif, en ce sens que, pour les produits de formule (1) ci-dessus, seul l'atome de brome en position 1 est substitué, et ceci en même en cas d'utilisation d'un fort excès stoëchiométrique d'hydrogène. Les rendements en dérivés monobromés sont élevés.

La réaction peut en outre être conduite dans une large gamme de pressions et de températures, et selon de nombreux modes de réalisation. Elle peut ainsi être conduite en masse ou en milieu solvant, et la catalyse peut être de type homogène, homogène supportée, ou hétérogène.

Dans tous les cas, il y a récupération et réutilisation du catalyseur, ce qui ajoute à l'économie du procédé. Elle peut être réalisée en batch, en semicontinu, en continu, en réacteur agité, ou en lit fixe ruisselant. Enfin, le procédé selon l'invention présente l'avantage de pouvoir être conduit directement, sans séparation ou purification préalable, sur le produit de réaction obtenu par bromation directe d'un dérivé non bromé du naphtalène substitué en position 2 par un radical OR, et contenant ainsi le dérivé dibromé de départ désiré.

Mais d'autres caractéristiques, aspects et avantages de l'invention apparaîtront encore plus clairement à la lecture de la description qui va suivre, et des exemples concrets mais non limitatifs destinés à l'illustrer.

La réaction catalytique impliquée dans le procédé selon l'invention peut être de type homogène ou hétérogène. Elle est dite homogène lorsque le catalyseur est soluble dans le milieu de réaction (système monophasique liquide) et hétérogène lorsque le catalyseur se présente sous une forme solide et insoluble dans ledit milieu (système au moins diphasique liquide/solide). Dans le cas d'une catalyse homogène, deux variantes sont possibles. Le milieu réactionnel peut être tout d'abord biphasique liquide/liquide, si le catalyseur est sous la forme d'un complexe hydrosoluble et le dérivé dibromé en solution organique non miscible à l'eau. Une telle variante est notamment décrite dans le brevet US 4 925 990 pour l'hydrogènation des aldéhydes $\alpha,\beta$ insaturées. Ce procédé permet de recycler facilement le catalyseur.

Selon la seconde variante, le milieu réactionnel peut être également monophasique avec une seule phase liquide ; dans ce cas, pour recycler le catalyseur, on précipite par refroidissement le dérivé monobromé obtenu, et le catalyseur est maintenu en solution. Cette solution catalytique est alors aisément réutilisable. Selon la présente invention, il est avantageux de se placer dans les conditions d'une catalyse hétérogène, notamment en raison du fait que la récupération ultérieure du catalyseur est ainsi grandement facilitée puisque pouvant être effectuée par des moyens simples, comme une filtration ou une décantation.

Les catalyseurs d'hydrodébromation utilisés dans la présente invention sont des catalyseurs connus en soi. Ils ont été décrits notamment dans les ouvrages suivants : "Catalytic hydrogenation over Platinium Metals", P.N. RYLANDER, Academic Press, 1967, et "Practical Catalytic Hydrogenation" (Technics and Applications), M. FREIFELDER, Wiley Intersciences, 1971, ces deux ouvrages étant cités ici à titre de références non limitatives. Leur fonction est de permettre la substitution d'un atome de brome porté par un carbone aromatique par un atome d'hydrogène. Ces catalyseurs ont généralement une phase active à base de métaux précieux tels qu'au moins l'un des éléments platine, palladium, rhodium, irridium, ruthénium ou osmiun. La phase active peut consister en un mélange de ces éléments. Selon l'invention, on utilise de préférence une phase active à base de rhodium ou de palladium.

Dans le cas d'une catalyse homogène, le ou les éléments catalytiquement actifs peuvent se présenter à l'état métallique ou sous la forme d'un sel, soluble dans le milieu de réaction.

Dans le cas d'une catalyse hétérogène, on utilise généralement des catalyseurs constitués d'une phase active déposée sur un support. Le support peut être du type substrat monolithique (nid d'abeilles ou autres), ou se présenter sous une forme divisée. Par forme divisée, on entend des produits pulvérulents (poudres) et également les articles obtenus par mise en forme de ces produits (billes, pastilles, boulettes, granulés, extrudés, agglomérés, et autres, de section circulaire, ovale, trilobée ou multilobée, pleine ou creuse).

Les supports de type billes, pastilles et autres, présentent l'avantage de conduire à des catalyseurs pouvant être séparés ultérieurement du milieu de réaction très rapidement par simple décantation. Les catalyseurs de type pulvérulent nécessitent généralement, pour leur séparation, une étape de filtration.

Ces catalyseurs supportés peuvent présenter une teneur en métaux précieux comprise entre 0,1 et 90% en poids par rapport au poids total du catalyseur, et généralement comprise entre 0,5 et 5% en poids.

A tire d'exemples de nature de support, on peut citer, pris seuls ou mélanges : les charbons actifs ; les oxydes tels que la silice, l'alumine, les aluminosilicates, l'oxyde de titane, l'oxyde de magnésium, l'oxyde de zirconium ; les zéolithes ; ou bien encore des céramiques telles que le carbure ou le nitrure de silicium, cette liste n'étant bien sûr nullement limitative.

Bien entendu, tous les supports précités sont choisis avec une surface spécifique convenant pour une application catalytique.

Les catalyseurs préférés pour la mise en oeuvre du procédé selon l'invention sont les catalyseurs constitués d'une phase catalytique au palladium et/ou rhodium déposée sur un support type charbon actif ou alumine pulvérulente.

Selon l'invention, la réaction peut être conduite en masse ou en milieu solvant. Dans un mode préférentiel de mise en oeuvre du procédé selon l'invention, on opère en milieu solvant. Il a été trouvé que le choix du solvant à utiliser revêtait une importance particulière, et que ce choix devait se limiter à des solvants organiques, et plus particulièrement à des solvants organiques acides.

Selon l'invention, on doit entendre par solvants organiques acides :

- soit des solvants organiques pratiques choisis parmi les acides carboxyliques, simples ou fonctionnalisés ;
- soit des solvants organiques aprotiques contenant au moins un acide organique ou inorganique.

En particulier, il a été trouvé que les solvants organiques protiques de type alcoolique ne convenaient pas pour la présente invention.

A titre d'exemples non limitatifs d'acides carboxyliques convenant comme solvant pour la présente invention, on peut citer l'acide méthanoïque, éthanoïque, propanoïque, butanoïque, et trifluoroacétique. Par acides carboxyliques, on entend bien entendu couvrir également les acides polycarboxyliques, simples ou fonctionnalisés, qui conviennent ici tout à fait.

A titre d'exemples non limitatifs de solvants organiques aprotiques convenables, on peut citer entre autres :

- les hydrocarbures aromatiques, tels que notamment le benzène et les alkylbenzènes (éthyl-, butyl-, propyl-benzène...), le toluène et les xylènes.
- les hydrocarbures paraffiniques et cycloparaffiniques, tels que notamment les alcanes en $C_5$ - $C_{20}$ (iso et n-pentane, hexane,...) ; les alkylalcanes (diméthyl-2,2 butane, diméthyl-2,3 butane, méthyl-2 pentane, méthyl-3 pentane, diméthyl-2,4 pentane, diméthyl-2,3 pentane, triméthyl-2,2,4 pentane, triméthyl-2,3,4 pentane, méthyle-3 héxane, triméthyl- 2,2,5 héxane,...) ; les cycloalcanes (cyclopentane, cyclohexane,...) ; les alkylcycloalcanes (méthylcyclopentane, diméthyl-1,1 cyclopentane, diméthyl-1,2 et 1,3 cyclopentane, méthylcyclohexane, éthylcyclohexane, isopropylcyclohexane,...).
- les hydrocarbures halogénés, en particulier fluorés et chlorés, de composés paraffiniques, cycloparaffiniques, et aromatiques tels que par exemple mentionnés ci-dessus, et notamment le dichlorométhane, le dichloro -1,2 éthane et le chlorobenzène.
- les esters, tels que notamment les acétates et les benzoates, en particulier d'alkyle, comme par exemple l'acétate d'éthyle et le benzoate de méthyle.
- les ethers, tels que notamment le diméthoxyéthane.
- les amides, tels que notamment la N - méthylpyrolidone.

Bien entendu, il est également tout à fait possible, dans le cadre de la présente invention, d'utiliser à titre de solvant soit des mélanges d'acides carboxyliques, soit des mélanges de solvants organiques aprotiques, soit des mélanges entre des acides carboxyliques et des solvants organiques aprotiques.

A titre d'exemples non limitatifs d'acides pouvant être contenus, seuls ou en mélanges, dans les solvants organiques aprotiques tels que susmentionnés, on peut citer :

- parmi les acides inorganiques : l'acide nitrique, l'acide phosphorique, l'acide sulfurique, les acides halogéniques, tels que l'acide chlorhydrique ou l'acide bromhydrique ;
- parmi les acides organiques : les acides carboxyliques susmentionnés, l'acide méthanesulfonique, triflique, éthanesulfonique ou benzéne-sulfonique.

La quantité d'acide, en mole, qui est contenue dans le solvant organique aprotique est généralement comprise entre 0,1 et 5 fois la quantité en mole de dérivés dibromés engagée, de préférence entre 0,8 et 2 fois cette quantité.

La proportion de catalyseur à utiliser n'est pas critique et peut varier dans de larges limites ; généralement on utilise de 0,01% à 50% en poids de catalyseur par rapport au dérivé dibromé engagé, et de préférence entre 0,1 et 10% en poids.

La quantité d'hydrogène à utiliser peut également varier dans de larges limites ; elle doit néanmoins correspondre au minimum à la quantité stoëchiométrique nécessaire pour permettre la substitution complète de la moitié des atomes de brome qui ont apportés sous la forme du composé dibromé initial. Il n'y a pas de quantité limite supérieure à respecter.

Selon l'invention, l'hydrogène est de préférence utilisé sous une forme gazeuse moléculaire ($H_2$). On peut néanmoins également utiliser de l'hydrogène naissant, c'est à dire de l'hydrogène formé in situ dans le milieu de réaction par décomposition d'un composé précurseur tel qu'un formiate ou l'acide formique.

Les températures mises en oeuvre pour conduire la réaction peuvent varier dans de très larges limites. Dans le cas d'une réaction en masse, elles varient de la température de fusion du dérivé dibromé à une température n'excédant pas le point de décomposition dudit dérivé et/ou du produit de réaction.

Dans le cas d'une réaction conduite en milieu solvant, on peut opérer de la température ambiante jusqu'à, théoriquement, la température d'ébullition du solvant utilisé, en veillant toutefois là encore à ne pas excéder des températures où le dérivé débromé et/ou le produit de réaction pourraient se décomposer ; dans la pratique, on travaille généralement à des températures comprises entre 20°C et 200°C, et de préférence entre 50 et 150°C.

La réaction peut être conduite soit à pression atmosphérique dans un réacteur type ouvert, ou lit fixe ruisselant, dans lequel on fait barboter un courant continu d'hydrogène, soit, de préférence, sous pression autogène dans un réacteur fermé, du type autoclave, contenant une atmosphère d'hydrogène. Dans ce dernier cas, la pression en hydrogène peut aller de 1 à 50 bars, et de préférence de 5 à 20 bars.

La réaction est de préférence conduite sous agitation, et ceci généralement jusqu'à disparition complète ou quasi-complète du dérivé dibromé du naphtalène introduit comme réactif.

En fin de réaction, on sépare du milieu réactionnel le dérivé monobromé obtenu, et ceci par tout moyen connu en soi, tel que par exemple filtration, décantation, centrifugation, extraction ou distillation. Selon que le procédé a été conduit en phase homogène ou hétérogène, en masse ou en milieu solvant, la récupération du dérivé monobromé peut nécessiter la mise en oeuvre d'une ou de plusieurs des opérations de séparation susmentionnées. Ainsi, par exemple, dans le cas d'une réaction conduite en phase hétérogène et en milieu solvant, on procédera tout d'abord à la récupération des catalyseurs, notamment par filtration ou décantation, puis à la séparation du dérivé monobromé et de la phase solvante organique, par exemple par extraction à l'eau ou distillation.

Les catalyseurs et/ou les solvants ainsi récupérés, après éventuellement purification, peuvent alors être recyclés en tête du procédé.

Le dérivé monobromé récupéré peut, quant à lui, subir des étapes complémentaires de purification, si nécessaire.

On notera que le procédé selon l'invention s'applique particulièrement bien aux dérivés dibromés de départ pour lesquels R représente l'hydrogène ou une radical alkyle, en vue d'obtenir les dérivés monobromés en position 6 correspondants.

Des exemples illustrant l'invention vont maintenant être donnés.

Dans ces exemples, TT désigne le taux de transformation, c'est à dire le rapport :

$$\frac{\text{quantité en mole dérivé dibromé transformée}}{\text{quantité en mole de dérivé dibromé introduite}} \text{ X } 100$$

RR désigne le Rendement de la Réaction vis à vis d'un produit de réaction donné, c'est à dire le rapport :

$$\frac{\text{quantité en mole d'un produit formé}}{\text{quantité en mole de dérivé dibromé introduite}} \text{ X } 100$$

RT traduit la sélectivité de la réaction pour un produit de réaction donné, il est définit par le rapport RR/TT.

**Exemple 1**

Dans une ampoule en verre de 35 ml, on introduit : 9 mg d'un catalyseur d'hydrodébromation constitué de charbon actif revêtu, à raison de 5% en poids, de palladium ; 1,2 g de dibromo -1,6 naphtol-2 ; et 15 ml de dichloro-1,2 éthane contenant 0,6 g d'acide trifluorométhanesulfonique (solvant organique acide).

On introduit ensuite l'ampoule ouverte dans un autoclave (Hastellay C) de 125 ml. On purge alors deux fois l'autoclave par de l'azote à une pression de 10 bars.

On introduit ensuite dans l'autoclave 20 bars d'hydrogène, et on chauffe pendant 10 heures à 100°C, et ceci sous agitation.

Une analyse par dosage CPG (chromatographie en phase gazeuse) avec étalon interne donne les résultats suivants:

| | |
|---|---|
| | TT = 95% |
| Bromo-6 naphtol-2 | RT = 70% |

**Exemple 2**

Dans une ampoule en verre de 35 ml, on introduit : 9 mg d'un catalyseur constitué d'une poudre d'alumine revêtue, à raison de 5% en poids, de rhodium ; 15 ml de dichloro-1,2 éthane contenant 0,002 mole de HBr ; et 1,2 g de dibromo-1,6 naphtol-2.

On procède ensuite comme à l'exemple 1, sauf que le chauffage à 100° n'est maintenu que pendant 4 heures.

L'analyse CPG donne les résultats suivants:

| | |
|---|---|
| | TT = 39% |
| Bromo-6 naphtol-2 | RT = 90% |

**Exemple 3**

Dans une ampoule en verre de 35 ml, on introduit : 20 mg d'un catalyseur constitué de charbon actif revêtu, à raison de 2,5% en poids, de rhodium ; 1,2 g de dibromo-1,6 naphtol-2 ; et 15 ml d'acide acétique.

On procède ensuite comme à l'exemple 2.

L'analyse CPG donne les résultats suivants :

| | |
|---|---|
| | TT = 95% |
| Bromo-6 naphtol-2 | RT = 92% |

**Exemple 4**

Dans une ampoule en verre de 35 ml, on introduit : 10 mg d'acétate de palladium ; 1,2 g de dibromo-1,6 naphtol-2 ; et 15 ml d'acide acétique.

On procède ensuite comme à l'exemple 2.

L'analyse CPG donne les résultats suivants :

| | |
|---|---|
| | TT = 95% |
| Bromo-6 naphtol-2 | RT = 88% |

**Exemple 5**

Dans une ampoule en verre de 35 ml, on introduit : 45 mg d'un catalyseur constitué de charbon actif revêtu, à raison de 3% en poids, de palladium ; 1,3 g de dibromo-1,6 methoxy-2 naphtatène ; et 15 ml d'acide acétique.

On procède ensuite comme à l'exemple 2.

L'analyse HPLC donne les résultats suivants :

| | |
|---|---|
| | TT = 95% |
| Bromo-6 methoxy-2 naphtatène | RR = 57% |
| Bromo-6 naphtol-2 | RR = 28% |
| RT global en isomères 6 : | 89% |

**Exemple comparatif 6**

Dans une ampoule en verre de 35 ml, on introduit : 10 mg d'acétate de palladium ; 1,2 g de dibromo-1,6 naphtol-2 ; et de 15 ml d'éthanol.

On procède ensuite comme à l'exemple 2.

L'analyse CPG donne les résultats suivants :

| | |
|---|---|
| | TT = 95% |
| Bromo-1 naphtol-2 | RR = 17% |
| Naphtol-2 | RR = 66% |
| Bromo-6 naphtol-2 | RR = 0% |

**Exemple comparatif 7**

Dans une ampoule en verre de 35 ml, on introduit : 15 mg d'un catalyseur constitué de charbon actif revêtu, à raison de 3% en poids, de palladium ; 1,26 g de dibromo-1,6 methoxy-2 naphtalène ; et 15 ml de méthanol.

On procède ensuite comme à l'exemple 2.

L'analyse HPLC donne les résultats suivants :

| | TT = 95% |
|---|---|
| Bromo-1 methoxy-2 naphtalène | RR = 81% |
| Bromo-6 methoxy-2 naphtalène | RR = 0% |

## Revendications

1. Procédé de débromation de dérivés dibromés du naphtalène, caractérisé par le fait que l'on fait réagir, en masse ou dans un solvant organique acide, en présence d'un catalyseur d'hydrodébromation, (i) un dérivé dibromé du naphtalène de formule :

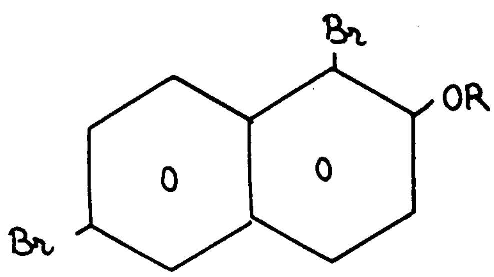

(1)

dans laquelle R représente l'hydrogène, un groupement alkyle, aryle, aralkyle ou

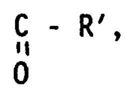

R′ étant un radical alkyle, avec (ii) de l'hydrogène moléculaire ou un composé susceptible de générer, dans le milieu de réaction, de l'hydrogène naissant.

2. Procédé selon la revendication 1, caractérisé en ce que R est l'hydrogène ou un groupement alkyle.

3. Procédé selon l'une des revendications 1 et 2, caractérisé en ce que ladite réaction est opérée en masse.

4. Procédé selon l'une des revendications 1 et 2, caractérisé en ce que ladite réaction est opérée dans un solvant organique acide.

5. Procédé selon la revendication 4, caractérisé en ce que ledit solvant organique acide est un acide carboxylique ou polycarboxylique, simple ou fonctionnalisé.

6. Procédé selon la revendication 4, caractérisé en ce que ledit solvant organique acide est un solvant organique aprotique contenant un acide organique ou inorganique.

7. Procédé selon la revendication 6, caractérisé en ce que ledit solvant organique aprotique est choisi parmi les hydrocarbures aromatiques, les hydrocarbures paraffiniques et cycloparaffiniques, les hydrocarbures halogénés, les esters, les ethers et les amides.

8. Procédé selon l'une quelconque des revendications 6 et 7, caractérisé en ce que ledit acide organique est choisi parmi les acides carboxyliques ou polycarboxyliques, simples ou fonctionnalisés, l'acide méthane - ou éthane - ou benzène - sulfonique, et l'acide triflique.

9. Procédé selon l'une quelconque des revendications 6 et 7, caractérisé en ce que ledit acide inorganique est choisi parmi l'acide nitrique, sulfurique, phosphorique, et les acides halogéniques.

**10.** Procédé selon l'une quelconque des revendications 6 à 9, caractérisé en ce que la quantité, en mole, dudit acide organique ou inorganique est comprise entre 0,1 et 5 fois la quantité en mole de dérivés dibromés.

**11.** Procédé selon la revendication 10, caractérisé en ce que ladite quantité est comprise entre 0,8 et 2 fois la quantité de dérivés dibromés.

**12.** Procédé selon l'une quelconque des revendications précédentes, caractérisé en ce que ledit catalyseur d'hydrodébromation présente une phase active à base d'au moins l'un des éléments platine, palladium, rhodium, irridium, ruthénium et osmium.

**13.** Procédé selon la revendication 12, caractérisé en ce que ladite phase active est déposée sur un support.

**14.** Procédé selon la revendicatin 13, caractérisé en ce que ledit support se présente sous une forme divisée.

**15.** Procédé selon la revendicatin 14, caractérisé en ce que ledit support est du charbon actif, un oxyde, une zéolithe ou une céramique.

**16.** Procédé selon la revendication 15, caractérisé en ce que ledit support est du charbon actif ou de l'alumine.

**17.** procédé selon l'une quelconque des revendications précédentes, caractérisé en ce que l'on utilise de l'hydrogène moléculaire $H_2$.

**18.** Procédé selon l'une quelconque des revendications 1 à 16, caractérisé en ce que ledit composé générant de l'hydrogène naissant est l'acide formique ou un formiate.

**19.** Procédé selon l'une quelconque des revendications précédentes, caractérisé en ce que l'on travaille sous pression.

**20.** Procédé selon l'une quelconque des revendications précédentes, caractérisé en ce qu'il est mis en oeuvre sur le produit de la bromation directe d'un dérivé du naphtalène de formule :

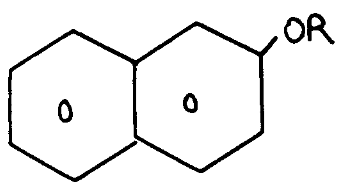

dans laquelle R a la signification donnée ci-avant.

## Claims

**1.** Process for the debromination of dibrominated naphthalene derivatives, characterized in that (i) a dibrominated naphthalene derivative of formula:

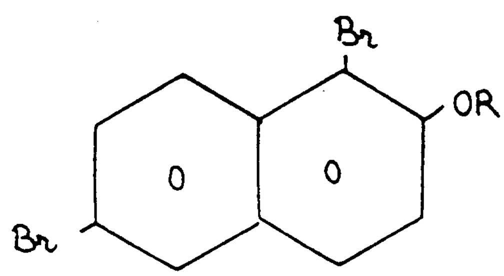

in which R represents hydrogen or an alkyl, aryl, aralkyl or

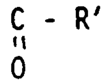

group, R' being an alkyl radical, is reacted, in bulk or in an acidic organic solvent, in the presence of a hydrodebromination catalyst, with (ii) molecular hydrogen or a compound capable of generating nascent hydrogen in the reaction mixture.

**2.** Process according to Claim 1, characterized in that R is hydrogen or an alkyl group.

**3.** Process according to either of Claims 1 and 2, characterized in that the said reaction is carried out in bulk.

**4.** Process according to either of Claims 1 and 2, characterized in that the said reaction is carried out in an acidic organic solvent.

**5.** Process according to Claim 4, characterized in that the said acidic organic solvent is a simple or functionalized carboxylic or polycarboxylic acid.

**6.** Process according to Claim 4, characterized in that the said acidic organic solvent is an aprotic organic solvent containing an organic or inorganic acid.

**7.** Process according to Claim 6, characterized in that the said aprotic organic solvent is chosen from aromatic hydrocarbons, paraffinic and cycloparaffinic hydrocarbons, halogenated hydrocarbons, esters, ethers and amides.

**8.** Process according to either of Claims 6 and 7, characterized in that the said organic acid is chosen from simple or functionalized carboxylic or polycarboxylic acids, methane- or ethane- or benzenesulphonic acid, and triflic acid.

**9.** Process according to either of Claims 6 and 7, characterized in that the said inorganic acid is chosen from nitric, sulphuric or phosphoric acid and halo acids.

**10.** Process according to any one of Claims 6 to 9, characterized in that the quantity, in moles, of the said organic or inorganic acid is between 0.1 and 5 times the quantity in moles of dibrominated derivatives.

**11.** Process according to Claim 10, characterized in that the said quantity is between 0.8 and 2 times the quantity of dibrominated derivatives.

**12.** Process according to any one of the preceding claims, characterized in that the said hydrodebromination catalyst has an active phase based on at least one of the elements platinum, palladium, rhodium, iridium, ruthenium and osmium.

**13.** Process according to Claim 12, characterized in that the said active phase is deposited on a support.

**14.** Process according to Claim 13, characterized in that the said support is provided in a divided form.

**15.** Process according to Claim 14, characterized in that the said support is active charcoal, an oxide, a zeolite or a ceramic.

**16.** Process according to Claim 15, characterized in that the said support is active charcoal or alumina.

**17.** Process according to any one of the preceding claims, characterized in that molecular hydrogen $H_2$ is used.

**18.** Process according to any one of Claims 1 to 16, characterized in that the said compound generating nascent hydrogen is formic acid or a formate.

**19.** Process according to any one of the preceding claims, characterized in that the work is carried out under pressure.

**20.** Process according to any one of the preceding claims, characterized in that it is used on the product of the direct bromination of a naphthalene derivative of formula:

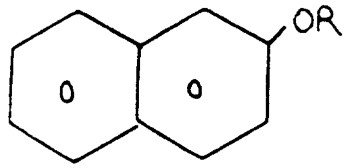

in which R has the meaning given above.

**Patentansprüche**

**1.** Verfahren zur Entbromung von dibromierten Naphthalinderivaten, dadurch **gekennzeichnet**, daß in Anwesenheit eines Hydroentbromungskatalysators in einem organischen Lösungsmittel oder auf die Masse selbst

(i) ein dibromiertes Naphthalinderivat gemäß der Formel:

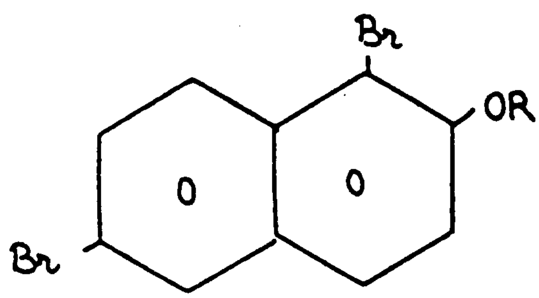

(1)

worin
R Wasserstoff, einen Alkyl-, Aryl- oder Aralkylrest oder den Rest

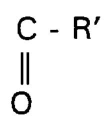

bedeutet, wobei R' einen Alkylrest darstellt, mit

(ii) molekularem Wasserstoff oder einer Verbindung zu dessen Erzeugung in dem Reaktionsmedium für naszierenden Wasserstoff zur Reaktion gebracht werden.

**2.** Verfahren nach Anspruch 1, dadurch **gekennzeichnet**, daß R Wasserstoff oder einen Alkylrest darstellt.

**3.** Verfahren nach einem der Ansprüche 1 und 2, dadurch **gekennzeichnet**, daß die Reaktion an der Masse durchgeführt wird.

**4.** Verfahren nach einem der Ansprüche 1 und 2, dadurch **gekennzeichnet**, daß die Reaktion in einem sauren organischen Lösungsmittel durchgeführt wird.

**5.** Verfahren nach Anspruch 4, dadurch **gekennzeichnet**, daß das saure organische Lösungsmittel eine einfache Carbon- oder Polycarbonsäure oder eine solche oder mit funktionellen Gruppen darstellt.

6. Verfahren nach Anspruch 4, dadurch **gekennzeichnet**, daß das saure organische Lösungsmittel ein aprotisches organisches Lösungsmittel mit einem Gehalt an einer organischen oder anorganischen Säure darstellt.

7. Verfahren nach Anspruch 6, dadurch **gekennzeichnet**, daß das aprotische organische Lösungsmittel aus aromatischen Kohlenwasserstoffen, paraffinischen und cycloparaffinischen Kohlenwasserstoffen, halogenierten Kohlenwasserstoffen, Estern, Ethern und Amiden ausgewählt wird.

8. Verfahren nach einem der Ansprüche 6 und 7, dadurch **gekennzeichnet**, daß die organische Säure unter einfachen Carbon- oder Polycarbonsäuren, die funktionelle Gruppen aufweisen können, der Methan- bzw. Ethan- bzw. Benzolsulfonsäure und der Trifluormethansulfonsäure (Triflat) ausgewählt werden.

9. Verfahren nach einem der Ansprüche 6 und 7, dadurch **gekennzeichnet**, daß die anorganische Säure aus Salpetersäure, Schwefelsäure, Phosphorsäure und den halogenierten Säuren ausgewählt wird.

10. Verfahren nach einem der Ansprüche 6 bis 9, dadurch **gekennzeichnet**, daß das Vielfache der Menge an der organischen oder anorganischen Säure, ausgedrückt in Mol, in einem Bereich zwischen 0,1 und 5 mal im Vergleich zur Menge an dibromierten Derivaten, ausgedrückt in Mol, beträgt.

11. Verfahren nach Anspruch 10, dadurch **gekennzeichnet**, daß das Vielfache deren Menge im Vergleich zur Menge an dibromierten Derivaten zwischen 0,8 und 2 beträgt.

12. Verfahren nach einem der vorstehenden Ansprüche, dadurch **gekennzeichnet**, daß der Hydroentbromungskatalysator in aktiver Phase auf Basis mindestens eines der Elemente Platin, Palladium, Rhodium, Iridium, Ruthenium und Osmium vorliegt.

13. Verfahren nach Anspruch 12, dadurch **gekennzeichnet**, daß die aktive Phase auf einem Träger aufgebracht ist.

14. Verfahren nach Anspruch 13, dadurch **gekennzeichnet**, daß der Träger in fein zerteilter Form vorliegt.

15. Verfahren nach Anspruch 14, dadurch **gekennzeichnet**, daß der Träger Aktivkohle, ein Oxid, ein Zeolith oder eine Keramik darstellt.

16. Verfahren nach Anspruch 15, dadurch **gekennzeichnet**, daß der Träger eine Aktivkohle oder eine Tonerde darstellt.

17. Verfahren nach einem der vorstehenden Ansprüche, dadurch **gekennzeichnet**, daß molekularer Wasserstoff in Form von $H_2$ verwendet wird.

18. Verfahren nach einem der Ansprüche 1 bis 16, dadurch **gekennzeichnet**, daß die Verbindung zur Erzeugung von naszierendem Wasserstoff die Ameisensäure oder ein Formiat darstellt.

19. Verfahren nach einem der vorstehenden Ansprüche, dadurch **gekennzeichnet**, daß unter Druck gearbeitet wird.

20. Verfahren nach einem der vorstehenden Ansprüche, dadurch **gekennzeichnet**, daß die Reaktion an dem direkten Bromierungsprodukt eines Naphthalinderivats gemäß der folgenden Formel stattfindet:

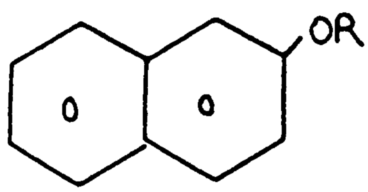

worin R die vorstehende Bedeutung aufweist.